# EUROPEAN PATENT APPLICATION

(11) **EP 4 233 925 A1**
(43) Date of publication of application: **30.08.2023**
(21) Application number: 22158238.0
(22) Date of filing: 23.02.2022
(51) Int. Cl.: A61L 27/36, A61L 27/44, A61L 27/38, A01K 61/70, A61K 35/614, B33Y 70/00

(54) **CORAL BIOINKS**

(71) Applicant: CELLINK Bioprinting AB, 412 77 Göteborg (SE); ZOAN NUAIL TEORANTA, Recess Galway (IE)
(72) Inventor: Redwan, Adel Itedal Namro, 44641 Skepplanda (SE); Kuzmenko, Volodymyr, 42542 Hisings Kärra (SE); Pernevik, Elin Astrid Margareta, 41261 Gothenburg (SE); Wann, Stephen, Recess (IE); Johnson, Martin, Recess (IE)
(74) Representative: HGF

(57) **Abstract**

A bioink is described. The bioink comprises coral particles and biocompatible polymer, wherein the concentration of coral particles in the bioink is 25 to 85 weight % of the total weight of the bioink.

## Description

### BACKGROUND

The present disclosure relates to bioinks comprising coral particles. The present disclosure also relates to a method of producing a coral scaffold, as well as to coral scaffolds that can be produced from the bioinks. The present disclosure also relates to a method of growing coral.

Many of the earth's coral reefs are coming increasingly under threat from, for example, climate change, ocean acidification, overfishing and pollution. Researchers are in the early stages of developing a range of methods to study, protect and restore the world's coral reefs. For instance, some recent restoration efforts have involved placing support structures in the sea. When coral larvae come into contact with such structures, they can settle in the pores or features of the structure and grow.

Coral has been shown to be biocompatible, osteoconductive, and biodegradable material. Although not osteoinductive or osteogenic, corals can allow cell attachment, growth, spreading and differentiation. When applied appropriately, coral scaffolds can be used as bone graft substitutes.

In 3D printing, a physical object is manufactured by a printing process, usually from a three-dimensional digital model. The process can involve laying down multiple layers of a material in succession to build the object in a layer-by-layer process. 3D printing can be used to produce objects of various shapes or configurations on-demand. Various types of 3D printing methods exist, including, for example, binder jetting, inkjet technology, powder bed fusion, material extrusion and sheet lamination.

Bioprinting can be used to fabricate functional tissue models for disease research purposes or for replacement of injured or diseased tissues in regenerative medicine, for drug screening instead of animal models of for biological assays. It is a relatively new approach that can provide high reproducibility and control over the fabricated constructs in an automated manner, potentially enabling high-throughput production. During bioprinting, a bioink may be used to create tissue constructs. The bioink can be partially crosslinked or stabilized, prior, during or immediately after bioprinting to generate the final shape, structure, and architecture of the designed construct.

### BRIEF DESCRIPTION OF FIGURES

Features of the present disclosure are described, by way of examples, with respect to the accompanying figures, in which:
Figures 1 and 2 depict schematic plan and sectional views of a 3D construct produced according to an example of the present disclosure;
Figure 3 shows the shear-thinning properties of bioinks comprising nanocellulose and alginate as biocompatible polymers and coral particles at coral particle loads of 6, 14, 25 and 30 weight %, respectively (see Example 1);
Figure 4 shows the multi-layered print fidelity that can be achieved using bioinks comprising nanocellulose and alginate as biocompatible polymers and coral particles at coral particle loads of 6, 14, 25 and 30 weight %, respectively (see Example 1); and
Figures 5, 6 and 7 show temperature dependent behavior, viscosity and print fidelity of bioinks comprising gelatin-methacrylate, alginate and xanthan gum, as biocompatible polymers, and coral particles at coral particle loads of 6 and 14 weight %, respectively (see Example 2).

### DETAILED DESCRIPTION

In the present disclosure, bioink formulations containing coral particles have been developed for the extrusion of coral scaffolds. To date, constructs formed using bioinks have lacked the stiffness and/or strength in compression that can be required for applications, such as scaffolds for bone tissue engineering applications or new coral growth. However, it has been found that, when coral particles are used in tailored amounts, they can interact with biocompatible polymer in the bioink to sustain shear thinning properties necessary for bioprinting of scaffolds with desirable shape fidelity. This is important, as increasing the proportion of coral relative to the proportion of biocompatible polymer would be expected to decrease the cohesiveness of the extruded structure and/or compromise the self-standing properties of the extruded part. In the present disclosure, it has been found that by controlling the amount of coral in the bioink, it is possible to achieve a bioink that can be extruded with high printing fidelity to maintain the integrity of the extruded part and with coral content that is high enough to provide functionality for intended applications such as bone tissue engineering and coral growth. Furthermore, coral particles may provide the resulting coral scaffold roughness, porosity and/or topography for promoting cell attachment and growth.

A first aspect of the present disclosure provides a first bioink comprising coral particles and biocompatible polymer, wherein the concentration of coral particles in the first bioink is 25 to 85 weight % of the total weight of the first bioink.

Preferably, the concentration of coral particles is 30 to 80 weight %, for example, 40 to 75 weight % or 50 to 70 weight % of the total weight of the first bioink.

According to a second aspect, there is provided a method of producing a coral scaffold. The method comprises producing a first portion of the scaffold by extrusion of the first bioink. The method also comprises crosslinking the biocompatible polymer.

According to a third aspect, there is provided a coral scaffold comprising a portion comprising coral and a crosslinked biocompatible polymer matrix, wherein the coral is present in an amount of 25 to 85 weight % of the portion.

The weight % of coral in the portion of the coral scaffold may be the same or different from than the weight % of coral particles in the first bioink, depending, for example, if any solvent is evaporated following the crosslinking. In some examples, the weight % of coral in the portion of the coral scaffold is the same or higher as the weight % of coral particles in the first bioink.

The concentration of coral particles in the first bioink may be maintained between 25 to 85 weight % to provide the extruded scaffold with desirable mechanical properties (e.g. stiffness and/or compressive strength), while maintaining the cohesiveness of the structure to ensure integrity of the extruded scaffold. These mechanical properties can provide the coral scaffold with utility in conservation and orthopaedic applications, where e.g. stiffness and compressive strength may be required. For example, to support new coral growth, a high degree of mechanical stiffness and/or compressive strength may be required to support coral larval settlement and the weight of the growing coral. Furthermore, for bone tissue engineering, high values of mechanical stiffness and/or compressive strength may be required during the period of healing. These mechanical properties can be achieved by tailoring the amount of coral in the first bioink from 25 to 80 weight %.

To facilitate bone healing and/or new coral formation, it may also be preferable to ensure that the coral particles are of a particular size. For example, the coral particles may have a particle size of at least 30 µm, preferably at least 32 or at least 35 µm, for instance from 30 µm to 200 µm or from 50 µm to 150 µm. Such larger particles (>30 µm) can preserve more of the surface and internal features of the coral skeleton. This can provide an improved environment for the attachment of, for example, bone cells or coral larvae.

The concentration of biocompatible polymer in the first bioink may be 1 to 75 weight % of the total weight of the first bioink. In some examples, the concentration of biocompatible polymer in the first bioink may be 5 to 50 weight % of the total weight of the first bioink.

Although the first bioink described above can include biological cells and/or biologically active molecules, cells and/or certain biologically active molecules may be sensitive to extrusion pressures during printing. As the concentration of coral particles in the bioink increases, higher extrusion pressures may be required during the printing process. High coral particle concentrations may also expose living cells or biological active molecules to increased abrasion, for example, during grinding of the bioink components. High coral particle concentrations, therefore, may impede the viability of living cells or sensitive bioactive molecules present in the bioink. In some embodiments, the first bioink may be substantially free from living biological cells.

In certain embodiments, it may nevertheless be desirable to incorporate biological cells and/or other biologically active molecules into the coral scaffold structure. Such cells and/or biologically active molecules can promote bone cell and/or coral larval growth by providing biochemical cues within the scaffold, which favours cell attachment and growth. To incorporate such components into the coral scaffold, it may be preferable to extrude portions of the coral scaffold with a bioink that is tailored to ensure the viability of such components.

Thus, according to a fourth aspect of the present disclosure, there is provided a second bioink comprising coral particles, biocompatible polymer and biological cells and/or biologically active molecules, wherein the concentration of coral particles in the bioink is 1 to 25 weight % of the total weight of the bioink.

Preferably, the second bioink comprises 3 to 25 weight %, for example, 6 to 14 weight % of coral particles.

The weight % of coral in the portion of the coral scaffold may be the same or different from the weight % of coral in the second bioink, depending, for example, if any solvent is evaporated following the crosslinking. In some examples, the weight % of coral in the portion of the coral scaffold may be the same or higher than the weight % of coral particles in the second bioink.

According to a fifth aspect, there is provided a method of producing a coral scaffold, said method comprising producing a second portion of the coral scaffold by extrusion of the second bioink. The method also comprises crosslinking the biocompatible polymer.

According to a sixth aspect, there is provided a coral scaffold comprising a portion comprising coral, biological cells and/or bioactive molecules in a crosslinked biocompatible polymer matrix, wherein the coral is present in an amount of 1 to 25 weight % of the portion.

Examples of biological cells include osteoblasts, stem cells, coral zygotes, coral planulae and zooxanthellae. In some embodiments, the biological cells may be isolated and cultured cell lines from coral organisms. Examples of biologically active molecules include growth factors, biomarkers and nutrients.

Preferable for the concentration of biological cells in the bioink composition to be maintained between 0 and 100 000 000 cells per gram of bioink. In some embodiments, the first bioink may be substantially free from living biological cells.

Although the second bioink may not be intended for forming weight-bearing portions of the coral scaffold, the presence of coral in the second bioink may nevertheless help to provide the coral scaffold with some rigidity and the physical topography for cell attachment. Coral can also help to improve the chemical microenvironment of potential cell settlement sites. Because of the biocompatibility of coral for coral larval and/or bone cell attachment, the coral particles in the second bioink may act as a carrier for the biological cells and/or the biologically active molecules. The combination of coral and biological cells/biologically active molecules, therefore, can improve bone cell attachment and/or coral larval settlement and growth on the extruded scaffold. However, as explained above, high concentrations of coral particles can give rise to high extrusion pressures that can be detrimental to the viability of cells post-extrusion. By maintaining the concentration of coral particles below 25 weight %, it is possible to preserve the viability of the cells and/or biologically active molecules post-extrusion, while benefiting from the use of coral as a carrier for biological cells and/or the biologically active molecules inclusion within the scaffold.

To maintain the viability of biological cells and biologically active molecules during preparation of the second bioink, it may be preferable to employ coral particles having a particle size of less than 35 µm, preferably less than 32 µm or less than 30 µm, for example, less than 25 µm. By using finer particles, the risk of damage, e.g., during any mixing and extrusion steps, may be reduced.

The biocompatible polymer may be present in the second bioink in a concentration of 1- 75 weight % of the total weight of the second bioink.

In some examples, it may be possible to use the second bioink to form selected portions of the coral scaffold, e.g., to coat selected portions of the coral scaffold. Accordingly, the coral scaffold may comprise a first portion formed from the first bioink with relatively high stiffness and/or compressive strength, and a second portion formed from the second bioink that is tailored to enhance cell attachment and growth. This can allow portions of the coral scaffold to be tailored for specific purposes.

Preferably, the method of producing the coral scaffold comprises producing a first portion of the scaffold by extrusion of the first bioink; and producing a second portion of the coral scaffold by extrusion of the second bioink. The method may further comprise crosslinking the biocompatible polymer in the first portion and/or second portion of the scaffold.

More preferably, the method comprises producing the first portion to provide a supporting structure of the scaffold, and which comprises producing the second portion to form a coating on at least part of the supporting structure, said coating providing a substrate for cell attachment and growth. The coating may be present on an external or on an internal surface of the supporting structure formed from the first bioink.

In some embodiments, there is provided a coral scaffold comprising a first portion comprising coral and a crosslinked biocompatible polymer matrix, the first portion comprising at least 25% by weight of coral based on the total weight of the first portion, and a second portion comprising coral, and biological cells and/or bioactive molecules in a crosslinked biocompatible polymer matrix, the second portion comprising at most 25% by weight of coral based on the total weight of the second portion.

In some embodiments, there is provided a coral scaffold comprising a first portion comprising coral and a crosslinked biocompatible polymer matrix, the first portion comprising at least 30% by weight of coral based on the total weight of the first portion, and a second portion comprising coral, and biological cells and/or bioactive molecules in a crosslinked biocompatible polymer matrix, the second portion comprising at most 20% by weight of coral based on the total weight of the second portion.

The coral scaffold may also be used for orthopaedic applications, for example, as bone graft substitutes. Coral is recognized as an osteoconductive biomaterial which can enhance bone healing and new bone formation when implanted in humans or animals (see, for example, I. Pountos et al., "Is there a role of coral bone substitutes in bone repair?", Injury, 2016, 47(12), pp. 2606-2613). Extrusion of coral-containing bioinks can allow scaffolds of different shapes to be made on demand. As explained above, whereas bioinks can generally lack the stiffness or strength in compression for applications such as bone tissue engineering, e.g., bone grafting or reconstruction, it has been found that, by increasing the concentration of coral in the bioink, a bioink can be produced that can nevertheless be extruded to produce parts with desirable mechanical properties (e.g. stiffness or compressive strength) for bone graft applications. Although the relative amount of biocompatible polymer in the bioink is decreased as a result of the increased coral concentration, crosslinking can nevertheless allow the coral particles to be supported within a crosslinked biocompatible polymer matrix. By controlling the concentration of coral particles in the bioink, it may be possible to increase the stiffness of the resulting extruded part, while maintaining cohesiveness of the structure to provide a self-standing scaffold.

In some applications, the first and second bioinks (separately or together) could be used to develop tissue models for research and testing purposes. For example, a bone tissue model, printed with, e.g., mesenchymal stem cells or osteoblast cells, could be used to assess the biocompatibility and osteoconductivity of new potential bone graft materials or the effect of drugs or growth factors on bone growth. In time this could replace or complement the need for animal testing for assessing new orthopaedic treatments.

The coral scaffolds described herein may also be used in a method of coral growth. Growing coral may be useful for a wide range of applications, including, for example, coral conservation, particularly where the coral scaffold is produced using coral particles derived from cultivated coral. In one embodiment, once produced, the coral scaffolds may be placed in a growth medium and coral growth may be cultured over the coral scaffolds. Surface topography is important for coral growth and, to date, coral conservation efforts have focused on providing the surface topography for promoting coral attachment and growth. Surprisingly, however, it has been found that the microenvironment of potential coral settlement sites can also have a significant influence on coral settlement and growth. Accordingly, while support structures have been constructed with the surface topography required for coral attachment, it has been found that the growth and viability of coral growth can be enhanced through the use of coral, even in devitalised form. Thus, according to a seventh aspect, there is provided a method of growing coral that comprises extruding a bioink comprising coral particles and biocompatible polymer to form a coral scaffold precursor, crosslinking the coral scaffold precursor to form the coral scaffold; positioning the coral scaffold in a growth medium, and culturing coral growth over the coral scaffold.

By producing the coral scaffold from coral particles, it is possible to produce a coral scaffold with desirable properties for supporting coral growth. For example, extrusion printing can be used to provide the coral scaffold with the physical topography required for desirable larval settlement and growth. Furthermore, by including a biocompatible polymer and biologically active molecules (e.g., growth factors, biomarkers, nutrients) in the ink, it is possible to support biological cells in the produced coral scaffold, such that these cells and/or active molecules can enhance coral settlement and growth. In some examples, biologically active molecules, such as growth factors, biomarkers and/or nutrients may be incorporated into hydrogel spherules, which may, in turn, be included in the bioink.

It has also been found that, by varying the concentration of coral particles in the bioink, mechanical properties, such as the mechanical stiffness of the coral scaffold may be varied. In some examples, the concentration of coral particles in scaffolds may range from 1 to 85 weight %, for example, 1 to 25 weight % or 25 to 80 weight %.

In some embodiments, it may be possible to construct different portions of the coral scaffold using bioinks with different compositions. For instance, for portions of the coral scaffold where enhanced stiffness is desired, the concentration of coral in the bioink may be increased to increase the stiffness or compressive strength of the resulting extruded portion. Such portions may be formed of bioink comprising 25 to 85 weight % coral particles. For instance, the first bioink described above may be used.

For portions of the coral scaffold where, for example, it is desirable for biological cells and/or biologically active molecules to be present, it may be desirable to reduce the concentration of coral particles in the ink, as high concentrations of coral may increase the extrusion pressures that would be required for printing. High extrusion pressures (and any associated temperature rises) can present an increased risk of damage to biological cells and/or biologically active molecules present in the ink. In portions of the coral scaffold that are formed using cell-containing bioinks, therefore, the coral particle concentration in the bioink may be 1 to 25 weight %. For example, the second bioink may be used.

To culture coral growth over the coral scaffold, the coral scaffold may be placed in a liquid medium. Where the liquid medium is, for example, seawater, coral zygotes and/or coral planulae present in the seawater may come into contact and attach to the coral scaffold for coral growth. In some examples, the coral scaffold is extruded such that the outer surface of the scaffold comprises a plurality of surface features for the settling and attachment of coral larvae (planulae). In some instances, the method of the present disclosure may further comprise introducing coral zygotes (fertilized eggs) and/or coral planulae (coral larvae) and/or zooxanthellae (coral-symbiotic single celled algae) into the liquid medium, for example, to promote coral growth. Alternatively or additionally, the bioink used to form the coral scaffold may further comprise at least one of coral zygotes, coral planulae, zooxanthellae, cells and/or growth factors. Accordingly, the resulting scaffold may incorporate at least one of coral zygotes, coral planulae, zooxanthellae, cells and/or growth factors. Alternatively or additionally, after extrusion, the resulting coral scaffold may be treated so that at least one of coral zygotes, coral planulae, zooxanthellae, cells and/or growth factors are incorporated or deposited onto the coral scaffold. In some examples, coral growth is cultured around the coral scaffold to conserve, construct or reconstruct a coral reef.

In some instances, it may be possible to print the coral scaffolds at or near the sites of deployment, e.g., to reduce the need for transporting large coral scaffolds over large distances.

In some applications, the 2 bioinks (separately or together) could be used to create a coral tissue model. For example, printed with coral cell lines it could be used to assess the toxicity of marine pollutants (including pharmacological pollutants) to coral organisms. The coral tissue model may be used to test the response of coral species to stressors or to study the biochemistry of the organism.

### Coral

The coral present in the bioink and/or coral scaffold may be a calcifying species of coral. Any suitable genera or species of coral may be grown. Examples of suitable genera include *Pocillopora, Montipora, Favites, Favia, Porites, Goniastrea, Acanthestrea, Acropora, Alvepora, Fungia, Galaxea, Hydnophora, Millepora, Seriatopora, Stylophora, Sinularia, Alcyoniidae* and/or any coral of the order *Scleractinia.* For example, suitable species include *Pocillopora damicornis* and *Montipora digitata.* Preferably, species that can be grown in captivity in a mesocosm are selected.

The coral may be grown in any suitable medium. The coral may be grown in captivity. The medium may be an aqueous medium. The aqueous medium may be a saline solution. Examples of suitable media include freshwater, seawater and mixtures thereof. In some examples, a mixture of seawater and freshwater may be employed. In other examples, the medium may comprise a saline solution formed by dissolving sodium chloride and, optionally, other salts in water. The water may be filtered prior to use. In some examples, the medium may be circulated or agitated. This circulation or agitation may simulate the circulation of water that coral may be exposed to in its natural environment.

The concentration of certain elements in the growth medium may also be controlled. For example, the growth medium may be dosed with solutions of one or more salts, for example, metal salts. Examples of suitable salts include salts of magnesium, sodium, calcium, zinc and strontium. One or more of these salts may be employed. In some instances, metal cations present in the salts may be incorporated into the structure of the growing coral and/or otherwise promote coral growth. In some examples, the method comprises controlling the concentration of one or more metal ions in the growth medium by monitoring the concentration of such metal ions and dosing metal salts into the growth medium to maintain metal ion concentrations within target levels. The metal salts (e.g., as metal salt solutions) dosed into the growth medium may be selected, such that the concentration of metal ions may be controlled without substantially altering pH, dKH or the overall concentration of bicarbonate and carbonate in the growth medium. For example, the coral may incorporate metal ions, for instance, metal ions selected from magnesium, calcium, strontium, zinc and mixtures thereof. Suitable salts include chlorides, for example, magnesium chloride, calcium chloride and strontium chloride. Other suitable salts include phosphates, for example, sodium phosphate and hydroxides, for example calcium hydroxide. In some instances, anions present in the salts may be incorporated into the structure of the growing coral, or otherwise promote coral growth. As an example, phosphate ions present in phosphate salts (e.g., sodium phosphate) may facilitate growth of the coral skeleton.

The growth medium may also be dosed with, for example, iodine. The coral may use iodine for the synthesis of pigments, which may allow them to adapt to varying light conditions and provide their tissue with protection from UV radiation. The amount of iodine in the growth medium may be controlled within prescribed limits. In some examples, it may be possible to dose iron salts in the growth medium. It has been found, however, that, in some circumstances, iron can increase the rate of nitrification.

The amount of salt(s) and other additives added to the growth medium may be controlled within narrow limits. Accordingly, solutions of the salts/additives may be dosed at predetermined amounts into the growth medium.

During the period of coral growth, the temperature of the growth medium may be controlled. Suitable temperatures range from 10 to 32 degrees Celsius, preferably 19 to 32 degrees Celsius. Preferably, the temperatures are controlled within 20-27 degrees Celsius, more preferably at about 25 degrees Celsius.

The growth medium may also be subjected to a controlled amount of light on a pre-determined cycle as required. The growth medium may be irradiated with light for 4 to 20 hours a day, for example, 6 to 15 hours a day, preferably 8 to 12 hours a day.

It may take at least one month, for example, two to six months before coral is ready for harvesting/collection. In some instances, it may take three to four months before the coral is ready for harvesting. In some examples, the coral may be considered to be ready for harvesting once it reaches at least 150%, preferably, at least 180% or at least 200% of its original volume. Once ready for harvest, the coral may be collected using known methods.

The coral may be grown under suitable conditions to impart desirable characteristics to the coral for its end use. For example, for bone graft applications, the coral may be grown to incorporate e.g. minerals that facilitate wound healing.

### Devitalisation

The collected coral may be devitalised using any suitable method. For example, the coral may be devitalised by treatment with an oxidizing agent, for instance, hypochlorite (e.g., sodium hypochlorite). In one example, the coral may be treated with a solution of hypochlorite for a predetermined length of time. Suitable time periods range from 3 to 50 hours, for example, 5 to 40 hours. If required, the devitalised coral may then be rinsed with water (e.g., deionized water). The collected coral may be dried. Drying may be performed at elevated temperatures. Suitable temperatures range from 50 to 190 degrees Celsius, for instance, 80 to 100 degrees Celsius. In some instances, the collected coral may be subjected to depyrogenation. The devitalised coral may then be sized using any suitable technique.

In some examples, sizing may be carried out by crushing or milling. Any suitable crushing or milling technique may be used. For instance, the devitalised coral may be milled.

The coral particles may have a particle size of less than 1000 µm.

### Biocompatible Polymer

Any suitable biocompatible polymer may be used in the bioinks described herein. The polymer may be selected to provide the rheological properties for printing (e.g., shear-thinning) by extrusion, as well as to contribute to the structural integrity to the resulting coral scaffold, for example, after printing. The polymer is crosslinkable to provide the coral scaffold with desired mechanical properties post-extrusion.

Where the bioink includes biological cells, it may be desirable for the polymer to maintain a desired level of hydration to support the cells. The polymer may be a hydrogel.

Suitable biocompatible polymers include synthetic and natural polymers. The biocompatible polymer may be a biopolymer. The biopolymer may be a polysaccharide, oligosaccharide, lactones, lactates and/or protein. Examples of suitable polysaccharides include cellulose, alginate, gellan gum, agarose, xanthan gum, gellan gum and hyaluronic acid. Examples of suitable proteins include gelatin, collagen and fibrin/fibrinogen. Examples of suitable lactones or lactates include PCL, PLA, PLGA. A suitable oligosaccharide is chitosan.

In some examples, the biopolymer may be selected from cellulose, alginates, hyaluronic acid, agarose, chitosan, fibrin, collagen and gelatin.

In some examples, at least two biopolymers are present in the bioink and/or coral scaffold. A combination of biopolymers may be desirable to provide the balance of properties required, e.g., for printability and crosslinking. In some examples, two or three biopolymers may be present in combination. Where a combination of biopolymers is employed, one or more may be selected to improve printability, while the remainder may be crosslinkable biopolymers employed to allow curing of the extruded product.

In some examples, the bioink may comprise at least one crosslinkable biopolymer. Crosslinking may occur by any suitable mechanism. The crosslinkable polymer may be an alginate. For example, where an alginate is used, the alginate may be crosslinked by the addition of divalent ions, for example, calcium and/or strontium ions.

In some instances, the crosslinkable biopolymer may include a crosslinkable moiety, for example, a methacrylate and/or acrylate moiety. In some examples, the crosslinkable biopolymer may be an acrylated alginate, acrylated collagen and/or acrylated gelatin. In some examples, the crosslinkable biopolymer may be selected from methacrylated alginate, methacrylated collagen and/or methacrylated gelatin.

In some examples, the bioink may comprise at least one crosslinkable biopolymer and a nanocellulose, xanthan gum or gellan gum. The nanocellulose, xanthan gum or gellan gum may be included to improve the printability of the bioink.

In some examples, the bioink may comprise at least two crosslinkable biopolymers and a nanocellulose, xanthan gum or gellan gum. The nanocellulose, xanthan gum or gellan gum may be included to improve the printability of the bioink.

In some examples, the bioink may comprise one or two crosslinkable biopolymers and a nanocellulose, xanthan gum or gellan gum.

In some examples, the bioink may comprise an alginate and a nanocellulose and/or xanthan gum. For instance, the bioink may comprise alginate and nanocellulose. Where the bioink comprises alginate and nanocellulose, the weight ratio of nanocellulose to alginate may be at least 1:1, preferably at least 2:1, for example, at least 3:1 or at least 4:1. The alginate may be crosslinked by ionic crosslinking using multivalent cations, e.g., calcium ions. The nanocellulose may be included to improve the printability of the bioink.

In some examples, the bioink may comprise a methacrylated alginate, methacrylated collagen, methacrylated gelatin, gelatin, and/or alginate. A photoinitiator may additionally be present. A nanocellulose and/or xanthan gum may optionally be present. In one example, the bioink comprises a methacrylated gelatin, a photoinitiator and alginate. The methacrylated gelatin may be photocrosslinked in the presence of a photoinitiator, while the alginate may be crosslinked via an ionic mechanism. The nanocellulose and/or xanthan gum may be included to improve the printability of the bioink. In one example, the bioink may comprise methacrylated gelatin, xanthan gum and alginate. The ratio of methacrylated gelatin to alginate may be at least 1:1, preferably, at least 2:1, at least 3:1, at least 4:1, for example 5:1. The ratio of methacrylated gelatin to xanthan gum may be at least 1:1, preferably at least 2:1, at least 3:1.

In some examples, biopolymer is present in the bioink in an amount of up to 75 weight % based on the total weight of the bioink.

### First Bioink

As discussed above, the first bioink comprises coral particles and biocompatible polymer, wherein the concentration of coral particles in the first bioink is 25 to 85 weight % of the total weight of the first bioink.

Preferably, the concentration of coral particles is 30 to 80 weight %, for example, 40 to 75 weight % of the total weight of the first bioink.

As discussed above, the amount of coral in the first bioink is relatively high to provide the scaffold with desirable mechanical properties (e.g. stiffness and/or compressive strength), while maintaining the cohesiveness of the structure to ensure integrity and/or self-standing nature of the scaffold.

The coral particles may be derived from any suitable coral species, such as the coral species discussed above. Examples include *Pocillopora, Montipora, Favites, Favia, Porites, Goniastrea, Acanthestrea, Acropora, Alvepora, Fungia, Galaxea, Hydnophora, Millepora, Seriatopora, Stylophora, Sinularia, Alcyoniidae* and/or any coral of the order *Scleractinia.* For example, suitable species include *Pocillopora damicornis* and *Montipora digitata.* Preferably, species that can be grown in captivity in a mesocosm are selected.

The coral particles in the first bioink may have any suitable size. For example, the coral particles may have particle sizes less than 1000 µm. In some examples, the coral particles may have a particle size of at least 30 µm. In some examples, the coral particles may have a mean particle size of at least 30 µm. Such larger particles (e.g., >30 micron) can preserve more of the surface and internal features of the coral skeleton. This can provide an improved environment for the attachment of, for example, bone cells or coral larvae.

Any method of measuring particle size may be used. For example, the particle size distribution of a sample may be determined by sieving. A suitable method is described under DIN 66165-2:2016-08 with sieves manufactured to ISO 3310-1:2016 and ASTM E11 can be used.

Suitable biocompatible polymers include synthetic and natural polymers. The biocompatible polymer may be a biopolymer. The biopolymer may be a polysaccharide, oligosaccharide and/or protein. Examples of suitable polysaccharides include cellulose, alginate, gellan gum, agarose, xanthan gum, gellan gum and hyaluronic acid. Examples of suitable proteins include gelatin, collagen and fibrin/fibrinogen. A suitable oligosaccharide is chitosan.

In some examples, the biopolymer may be selected from cellulose, alginates, hyaluronic acid, agarose, chitosan, fibrin, collagen and gelatin.

In some examples, at least two biopolymers may be present in the bioink and/or coral scaffold. A combination of biopolymers may be desirable to provide the balance of properties required, for e.g., printability and crosslinking. In some examples, two or three biopolymers may be present in combination. Where a combination of biopolymers is employed, one or more may be selected to improve printability, while the remainder may be crosslinkable biopolymers employed to allow curing of the extruded product.

In some examples, the bioink may comprise at least one crosslinkable biopolymer. The crosslinkable polymer may be an alginate. Crosslinking may occur by any suitable mechanism. For example, where an alginate is used, the alginate may be crosslinked by the addition of multivalent ions (e.g., divalent ions), for example, calcium and/or strontium ions.

In some instances, the crosslinkable biopolymer may include a crosslinkable moiety, for example, a methacrylate and/or acrylate moiety. In some examples, the crosslinkable biopolymer may be an acrylated alginate, acrylated collagen and/or acrylated gelatin. In some examples, the crosslinkable biopolymer may be selected from methacrylated alginate, methacrylated collagen and/or methacrylated gelatin.

In some examples, the bioink may comprise at least one crosslinkable biopolymer and a nanocellulose, xanthan gum or gellan gum. The nanocellulose, xanthan gum or gellan gum may be included to improve the printability of the bioink.

In some examples, the bioink may comprise at least two crosslinkable biopolymers and a nanocellulose, xanthan gum or gellan gum. The nanocellulose, xanthan gum or gellan gum may be included to improve the printability of the bioink.

In some examples, the bioink may comprise one or two crosslinkable biopolymers and a nanocellulose, xanthan gum or gellan gum.

In some examples, the bioink may comprise an alginate and a nanocellulose and/or xanthan gum. For instance, the bioink may comprise alginate and nanocellulose. The alginate may be crosslinked by ionic crosslinking using multivalent cations, e.g., calcium ions. The nanocellulose may be included to improve the printability of the bioink.

In some examples, the bioink may comprise a methacrylated alginate, methacrylated collagen, methacrylated gelatin, a photoinitiator and/or an alginate or gelatin. A nanocellulose and/or xanthan gum may optionally be present. In one example, the bioink comprises a methacrylated gelatin, a photoinitiator and alginate. The methacrylated gelatin may be photocrosslinked in the presence of a photoinitiator, while the alginate may be crosslinked via an ionic mechanism. The nanocellulose and/or xanthan gum may be included to improve the printability of the bioink.

In some examples, at least two biopolymers are present in the bioink and/or coral scaffold. A combination of biopolymers may be desirable to provide the balance of properties required, for e.g., printability and crosslinking. In some examples, two or three biopolymers may be present in combination.

In some examples, the bioink may comprise at least one crosslinkable biopolymer. The crosslinkable polymer may be an alginate. Crosslinking may occur by any suitable mechanism. For example, where an alginate is used, the alginate may be crosslinked by the addition of multivalent ions, for example, calcium and/or strontium ions.

In some instances, the crosslinkable biopolymer may include a crosslinkable moiety, for example, a methacrylate and/or acrylate moiety. In some examples, the crosslinkable biopolymer may be an acrylated alginate, acrylated collagen and/or acrylated gelatin. In some examples, the crosslinkable biopolymer may be selected from methacrylated alginate, methacrylated collagen and/or methacrylated gelatin.

In some examples, the bioink may comprise at least one crosslinkable biopolymer and a polysaccharide, for example, cellulose, xanthan gum or gellan gum. Where cellulose is employed, the cellulose may take the form of cellulose nanofibrils.

In some examples, the bioink may comprise at least two crosslinkable biopolymers and polysaccharide, for example, cellulose, xanthan gum or gellan gum. Where cellulose is employed, the cellulose may take the form of cellulose nanofibrils.

In some examples, the bioink may comprise one or two crosslinkable biopolymers and a nanocellulose, xanthan gum or gellan gum.

In some examples, the bioink may comprise an alginate and a nanocellulose and/or xanthan gum.

Where the bioink comprises alginate and nanocellulose, the weight ratio of nanocellulose to alginate may be at least 1:1, preferably at least 2:1, for example, at least 3:1 or at least 4:1.

In some examples, the bioink may comprise a methacrylated alginate, methacrylated collagen, methacrylated gelatin, a photoinitiator and/or an alginate or gelatin. A nanocellulose and/or xanthan gum may optionally be present.

In one example, the bioink may comprise methacrylated gelatin, xanthan gum and alginate. The ratio of methacrylated gelatin to alginate may be at least 1:1, preferably, at least 2:1, at least 3:1, at least 4:1, for example 5:1. The ratio of methacrylated gelatin to xanthan gum may be at least 1:1, preferably at least 2:1, at least 3:1.

The first bioink may comprise an initiator to initiate the crosslinking reaction. In some examples, the initiator mat be a photoinitiator. Any photoinitiator that can initiate or induce polymerization of biopolymers, e.g., acrylated or methacrylated biopolymers, may be employed. Examples of the photoinitiator can include camphorquinone, benzoin methyl ether, 2-hydroxy-2-methyl- 1 -phenyl- l-propanone, diphenyl(2,4,6-trimethylbenzoyl)phosphine oxide, benzoin ethyl ether, benzophenone, 9,l0-anthraquinone, ethyl-4-N,N-dimethylaminobenzoate, diphenyliodonium chloride and derivatives thereof.

The first bioink may comprise additional molecules for adjusting osmotic pressure and pH in the bioink, for example, D-mannitol, HEPES, PBS.

The bioink shows a shear-thinning behavior at printing temperature with a viscosity of >5 kPa*s at close to zero shear and <100 Pa*s at 500/s shear.

In some examples, the first bioink may be extruded to form the supporting framework of the coral scaffold. This framework may have a porous structure with interconnectivity for cell migration and/or nutrient diffusion. In some examples, the bioink may be used to form a framework that can accommodate bioactive additives, such as growth factors or stem cells, that can stimulate cell growth. Such bioactive additives may be applied to the coral scaffold post-printing, and/or may be incorporated into the bioink formulation. In some examples, such additives may be applied by extruding a second bioink composition. In some examples, the bioink is extruded to form a structure that has shape and mechanical characteristics for performing a desired function post-implantation (e.g., in seawater or in the body).

### Second Bioink

As discussed above, the second bioink comprising coral particles, biocompatible polymer and biological cells and/or biologically active molecules, wherein the concentration of coral particles in the bioink is 1 to 25 weight % of the total weight of the bioink.

Preferably, the bioink comprises 3 to 25 weight %, for example, 6 to 14 weight % of coral particles.

Examples of biological cells include osteoblasts, stem cells, coral zygotes, coral planulae and zooxanthellae. Other examples of biological cells include isolated and cultured cell lines from coral organisms. Examples of biologically active molecules include growth factors, biomarkers and nutrients.

For orthopaedic applications, suitable biological cells include osteoblasts, stem cells, including but not limited to progenitor cells, MSC, iPSCs and ECs. Suitable biologically active molecules include but not limited to growth factors, peptides, lipids or extracellular matrix molecules.

For coral conservation applications, suitable biological cells include coral zygotes, coral planulae and zooxanthellae.

Where cells are present in the second bioink, they may be present at a concentration between 10 000 to 100 000 000 cells per gram of bioink.

Where biologically active molecules are present in the second bioink, they may be present at a concentration of 0.1 ng - 100 mg per gram bioink. For example, vitamins such as ascorbic acid and growth factors such as BMP2 might be present at 1 ng -500µg per gram bioink, small molecules such as dexamethasone or glycerol phosphate at 1ng to 10mg per gram bioink and peptides and proteins such as RGD, laminin or fibrinogen at 1 µg to 20mg per gram bioink.

Where biologically active molecules are present in the second bioink, they may be contained in hydrogel spherules dispersed in the second bioink.

In some examples, the second bioink may be extruded and selectively applied to produce portions of the coral scaffold. For example, where the first bioink is extruded to form a support portion of the coral scaffold, the second bioink may be extruded to coat at least a portion of the external and/or internal surfaces of the support portion to enhance the cell attachment and growth on the scaffold.

The coral particles may be derived from any suitable coral species, such as the coral species discussed above. Examples include *Pocillopora, Montipora, Favites, Favia, Porites, Goniastrea, Acanthestrea, Acropora, Alvepora, Fungia, Galaxea, Hydnophora, Millepora, Seriatopora, Stylophora, Sinularia, Alcyoniidae* and/or any coral of the order *Scleractinia.* For example, suitable species include *Pocillopora damicornis* and *Montipora digitata.* Preferably, species that can be grown in captivity in a mesocosm are selected.

The coral particles in the first bioink may have any suitable size. For example, the coral particles may have particle sizes less than 35 µm, for example, less than 30 µm. Smaller particle sizes may be favoured as small particles may impose less abrasive forces on the cells or biologically active molecules that may be present in the bioink, e.g., during mixing and extrusion.

Any method of measuring particle size may be used. For example, the particle size distribution of a sample may be determined by sieving. A suitable method is described under DIN 66165-2:2016-08 with sieves manufactured to ISO 3310-1:2016 and ASTM E11 can be used.

The second bioink may comprise 1 to 75 weight % biocompatible polymer, preferably 2.5 to 10 weight % biocompatible polymer. Suitable biocompatible polymers include synthetic and natural polymers. The biocompatible polymer may be a biopolymer. The biopolymer may be a polysaccharide, oligosaccharide and/or protein. Examples of suitable polysaccharides include cellulose, alginate, gellan gum, agarose, xanthan gum, gellan gum and hyaluronic acid. Examples of suitable proteins include gelatin, collagen and fibrin/fibrinogen. A suitable oligosaccharide is chitosan.

In some examples, the biopolymer may be selected from cellulose, alginates, hyaluronic acid, agarose, chitosan, fibrin, collagen and gelatin.

In some examples, at least two biopolymers are present in the bioink and/or coral scaffold. A combination of biopolymers may be desirable to provide the balance of properties required, for e.g., printability and crosslinking. In some examples, two or three biopolymers may be present in combination. Where a combination of biopolymers is employed, one or more may be selected to improve printability, while the remainder may be crosslinkable biopolymers employed to allow curing of the extruded product.

In some examples, the bioink may comprise at least one crosslinkable biopolymer. The crosslinkable polymer may be an alginate. Crosslinking may occur by any suitable mechanism. For example, where an alginate is used, the alginate may be crosslinked by the addition of multivalent ions, for example, calcium and/or strontium ions.

In some instances, the crosslinkable biopolymer may include a crosslinkable moiety, for example, a methacrylate and/or acrylate moiety. In some examples, the crosslinkable biopolymer may be an acrylated alginate, acrylated collagen and/or acrylated gelatin. In some examples, the crosslinkable biopolymer may be selected from methacrylated alginate, methacrylated collagen and/or methacrylated gelatin.

In some examples, the bioink may comprise at least one crosslinkable biopolymer and a nanocellulose, xanthan gum or gellan gum. The nanocellulose, xanthan gum or gellan gum may be included to improve the printability of the bioink.

In some examples, the bioink may comprise at least two crosslinkable biopolymers and a nanocellulose, xanthan gum or gellan gum. The nanocellulose, xanthan gum or gellan gum may be included to improve the printability of the bioink.

In some examples, the bioink may comprise one or two crosslinkable biopolymers and a nanocellulose, xanthan gum or gellan gum.

In some examples, the bioink may comprise an alginate and a nanocellulose and/or xanthan gum. For instance, the bioink may comprise alginate and nanocellulose. Where the bioink comprises alginate and nanocellulose, the weight ratio of nanocellulose to alginate may be at least 1:1, preferably at least 2:1, for example, at least 3:1 or at least 4:1.

The alginate may be crosslinked by ionic crosslinking using multivalent cations, e.g., calcium ions. The nanocellulose may be included to improve the printability of the bioink.

In some examples, the bioink may comprise a methacrylated alginate, methacrylated collagen, methacrylated gelatin, a photoinitiator and/or an alginate or gelatin. A nanocellulose and/or xanthan gum may optionally be present. In one example, the bioink comprises a methacrylated gelatin and alginate.

In one example, the bioink may comprise methacrylated gelatin, xanthan gum and alginate. The ratio of methacrylated gelatin to alginate may be at least 1:1, preferably, at least 2:1, at least 3:1, at least 4:1, for example 5:1. The ratio of methacrylated gelatin to xanthan gum may be at least 1:1, preferably at least 2:1, at least 3:1.

The methacrylated gelatin may be photocrosslinked in the presence of a photoinitiator, while the alginate may be crosslinked via an ionic mechanism. The nanocellulose and/or xanthan gum may be included to improve the printability of the bioink.

The second bioink may comprise an initiator to initiate the crosslinking reaction. In some examples, the initiator mat be a photoinitiator. Any photoinitiator that can initiate or induce polymerization of a biopolymer, e.g., acrylated or methacrylated biopolymer, may be employed. Examples of the photoinitiator can include camphorquinone, benzoin methyl ether, 2-hydroxy-2-methyl- 1 -phenyl- l-propanone, diphenyl(2,4,6-trimethylbenzoyl)phosphine oxide, benzoin ethyl ether, benzophenone, 9,l0-anthraquinone, ethyl-4-N,N-dimethylaminobenzoate, diphenyliodonium chloride and derivatives thereof.

The first bioink may comprise additional molecules for adjusting osmotic pressure and pH in the bioink, for example, D-mannitol, HEPES, PBS.

The formulation shows a shear-thinning behavior at printing temperature with a viscosity of >5kPa*s at close to zero shear and <30Pa*s at 500/s shear.

In some examples, the second bioink may be extruded over at least part of the supporting framework formed using the first bioink. As mentioned above, the framework may have a porous structure with interconnectivity for cell migration and/or nutrient diffusion. In some examples, the second bioink may be used to form a coating on at least part of the porous structure. The presence of biological cells and/or bioactive molecules can enhance cell attachment and proliferation (e.g., osteoblasts), and/or coral attachment and growth depending on the field of application.

### Coral Scaffold

As discussed above, the coral scaffold may be used for conservation applications to promote coral growth. For such applications, the coral scaffold may be extruded such that the outer surface of the scaffold comprises a plurality of surface features for the settling and attachment of coral larvae (planulae). The coral scaffold may further comprise at least one of coral zygotes, coral planulae, zooxanthellae, cells and/or growth factors. The cells may be isolated and cultured cell lines from coral organisms. The coral zygotes, coral planulae, zooxanthellae, cells and/or growth factors may be incorporated or deposited onto the coral scaffold as a post-extrusion step. Alternatively or additionally, the coral zygotes, coral planulae, zooxanthellae, cells and/or growth factors may be incorporated into the bioink used to form the coral scaffold.

The coral scaffold may also be used for bone graft applications. The coral scaffold may be printed as a coating, for example, on a bone implant (e.g., a metal bone implant). In some examples, the coral scaffold may be printed and used to fill voids in and around a bone implant (e.g., a metal bone implant). Preferably, the coral scaffold is printed with a supporting portion that is self-standing. This supporting portion is formed with the first bioink. Parts of the supporting portion may be coated with a coating formed with the second bioink.

In some examples, the coral scaffold may be used in combination with a different tissue scaffold. For example, the coral scaffold may be used in combination with tissue scaffold for supporting the growth of tissues, e.g., cartilage, skin or muscle tissue.

In some examples, the coral scaffold may incorporate protein, growth factor or cells (e.g., stem cells) to facilitate bone growth. Such factors and/or cells may be incorporated into the coral scaffold as a post-printing step, or may be incorporated in the ink used to produce the scaffold. The coral scaffold may comprise osteoinductive factors including human bone morphogenic protein 2 (h-BMP2). Other examples of factors include transforming growth factor β (TGF-β), platelet-derived growth factor (PDGF) and bioactive polypeptides. The protein, growth factor or cells may be present as a matrix within the porous structure of the coral scaffold. The coral scaffold may be used in combination with allograft tissue, and/or endogenous bone forming cells including mesenchymal stem cells, osteoprogenitor cells and osteoblasts as well as osteoinductive and angiogenic growth factors.

The coral scaffold may be suitable for use as an osteoconductive matrix. In some examples, the coral scaffold may be used as an osteoconductive matrix for cancellous bone tissue. In some examples, the coral scaffold may be used as an osteoconductive matrix for cortical bone tissue. The coral scaffold may be suitable for impaction grafting. The coral scaffold may be suitable for bone rebuilding, for example, following tumour removal and/or revision of artificial joints. The bone graft substitute may also be used for filling voids or other bone defects.

The coral scaffold may be used as a scaffold for bone growth in any bones in humans or other mammals including the spine, sternum, ribs, femur, tibia, fibula, talus, patella, ulna, radius, humerus, pelvis, scapula, skull, jawbone and teeth.

The coral scaffold may be used as an extender for an autograft or allograft bone. For example, the coral scaffold may be applied alongside allograft and/or allograft bone fragments.

The coral scaffold may be combined with bone marrow, e.g., prior or during the implantation process. In some examples, the bone graft substitute may further comprise bone marrow in combination with the coral particles.

### Figure 1

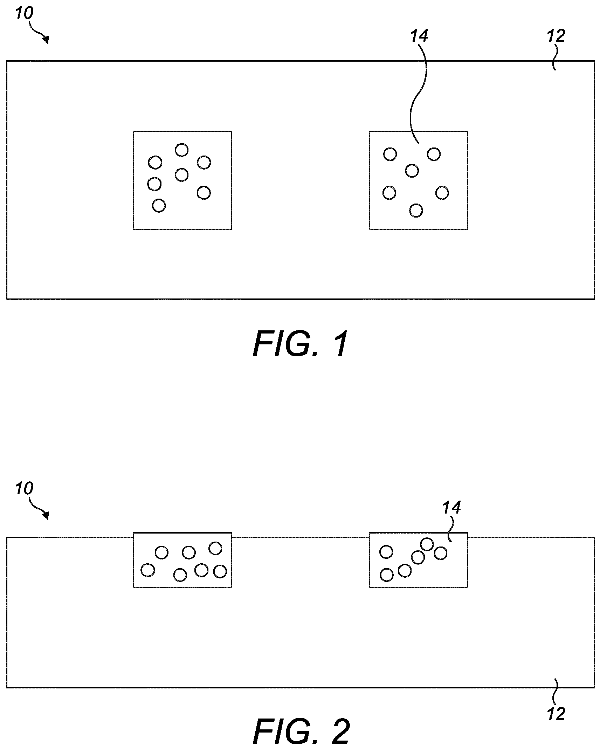

Figures 1 and 2 depict schematic plan and sectional views of a coral scaffold produced according to an example of the present disclosure.

The coral scaffold 10 comprises a first portion 12 and second portions 14. The first portion 12 comprises coral particles and a biocompatible polymer, wherein the concentration of coral particles in the first portion is, for example, 30 to 80 weight %.

The second portions 14 comprise coral particles, a biocompatible polymer and biological cells and/or biologically active molecules. The concentration of coral particles in the second portions is 1 to 25 weight %. The biological cells and/or biologically active molecules are shown schematically as circles.

The first portion 12 has greater mechanical strength than the second portion 14 and acts as a support around the second portions 14.

The first portion 12 is produced from a first bioink comprising coral particles and a biocompatible polymer, wherein the concentration of coral particles in the first bioink is, for example, 25 to 80 weight % of the total weight of the first-bioink. The first bioink may be extruded at high extrusion pressures.

The second portion 14 is produced using a second bioink comprising coral particles, a biocompatible polymer and biological cells and/or biologically active molecules, wherein the concentration of coral particles in the second bioink is 1 to 25 weight % of the total weight of the second bioink. The second bioink may be extruded under relatively lower extrusion pressures. This can reduce the risk of damage to the biological cells and/or biologically active molecules during the extrusion process. At the same time, biological cells and/or biologically active molecules may be incorporated into the structure of coral scaffold by extrusion printing, e.g., to promote osteogenesis or coral growth.

### Example 1

In this example, bioink A was prepared comprising 6 weight % *Pocillopora* coral particles (< 32 µm) and 2.5 - 5 weight % of a combination of alginate and cellulose nanofibrils in a 1:4 weight ratio.

Bioink B was prepared comprising 14 weight % *Pocillopora* coral particles (< 32 µm) and 2.5 - 5 weight % of a combination of alginate and cellulose nanofibrils in a 1:4 weight ratio.

Bioink C was prepared comprising 25 weight % *Pocillopora* coral particles (< 32 µm) and 2 - 5 weight % of a combination of alginate and cellulose nanofibrils in a 1:4 weight ratio.

Bioink D was prepared comprising 30 weight % *Pocillopora* coral particles (< 32 µm) and 2 - 5 weight % of a combination of alginate and cellulose nanofibrils in a 1:4 weight ratio.

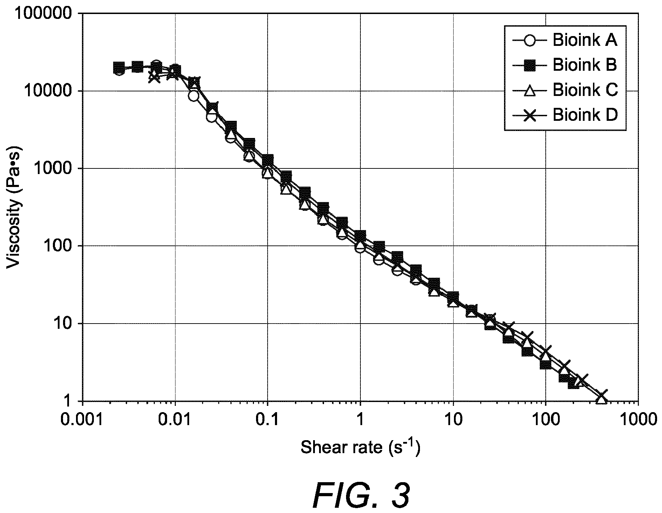

The shear-thinning profiles for Bioinks A to D were evaluated. These are shown in Figure 3. All inks were shown to show good shear thinning properties as viscosity decreases by orders of magnitude with increasing shear rates.

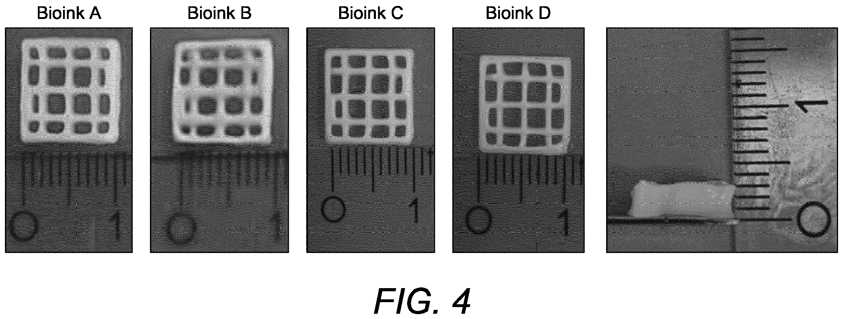

The bioinks were extruded to form grids at an extrusion pressure of 20 to 23 kPa (higher pressure for higher coral particle load) at a translational rate of 10 mm/s for a filament. The bioinks were then ionically crosslinked post-printing. As shown in Figure 4, bioinks A, B, C and D allowed printing multi-layered constructs with good shape fidelity, with good print definition along the internal and external walls of the grid. As shown in the far right image in Figure 4, the structure were also self-supporting.

Biological cells, e.g., osteoblasts, or biologically active molecules can be incorporated into bioinks A, B and C and printed in the presence of coral particles as the second bioink to provide the coral scaffold with some rigidity and the physical topography for biological cell attachment.

Bioinks C and D may be used as the first bioink to produce a coral scaffold or coral scaffold portion with the mechanical stiffness and/or compressive strength that may be required to support coral larval settlement and the weight of the growing coral. Alternatively, Bioinks C and D may be used as the first bioink to produce a coral scaffold or coral scaffold portion with the mechanical stiffness and/or compressive strength that may be required in bone tissue applications e.g. during bone healing.

### Example 2

In this example, bioink E was prepared comprising 6 weight % *Pocillopora* coral particles (< 30 µm) and 5 - 10 weight % of a combination of gelatin methacrylate (GeIMA), xanthan gum and alginate in a 10:3:2 weight ratio and lithium phenyl-2,4,6-trimethylbenzoylphosphinate (LAP) as a photoinitiator at 0.25 weight %.

Bioink F was prepared comprising 14 weight % *Pocillopora* coral particles (< 30 µm) and 5 - 10 weight % of a combination of gelatin methacrylate (GeIMA), xanthan gum and alginate in a 10:3:2 weight ratio, and LAP as photoinitiator at 0.25%.

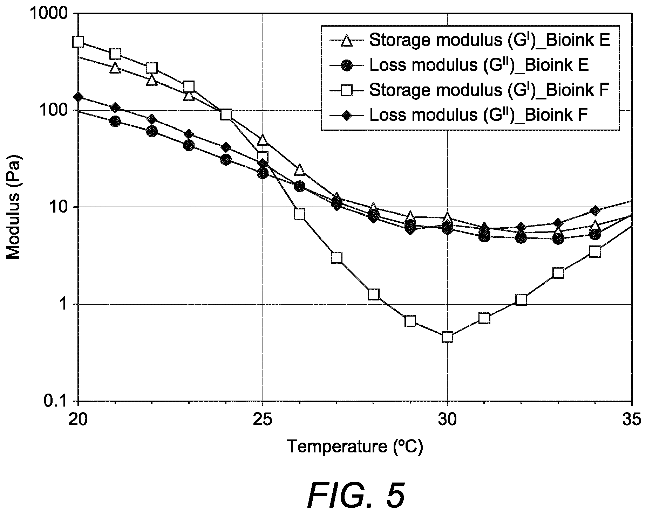

Rheological measurements (loss modulus and storage modulus) were performed with bioinks E and F over temperatures of 10 to 35 degrees Celsius to establish storage and loss modulus crossover temperature and relevant temperatures for evaluating flow properties during printing. These are shown in Figure 5.

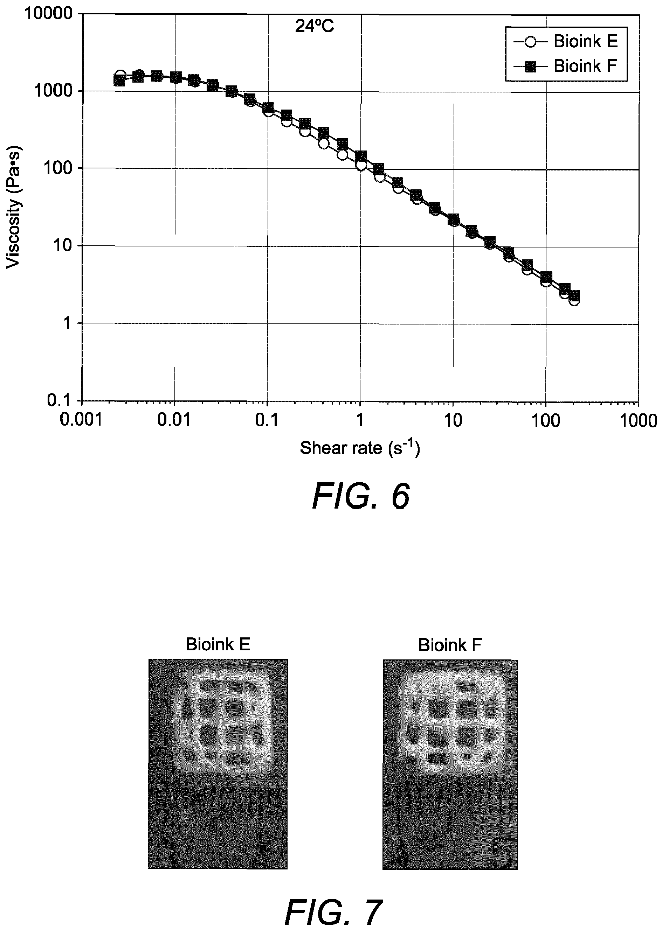

The shear-thinning profiles for both inks were evaluated at 24 degrees Celsius, close to the storage and loss modulus crossover. These are shown in Figure 6. Both inks were shown to show good shear thinning properties.

Bioink E was extruded to form a grid at extrusion pressures of 45 to 55 kPa with printhead temperatures of 25 to 26 degrees Celsius and print bed temperature of 15 degrees Celsius. The extrusion speed was 10 mm/s. The bioink was photocrosslinked using a 405 nm LED module with 43 mW/cm² to excite the LAP photoinitiator for 20 s post-printing.

Bioink F was extruded to form a grid at extrusion pressures of 35 to 47 kPa with printhead temperatures of 25 to 26 degrees Celsius and print bed temperature of 15 degrees Celsius. The extrusion speed was 10 mm/s. The bioink was photocrosslinked using a 405 nm LED module with 43 mW/cm² to excite the LAP photoinitiator for 20 s post-printing.

As shown in Figure 7, bioink E (left image) and bioink F (right image) were found to print with good shape fidelity. Though, the shape fidelity was better in Example 1, for Bioinks E and F it can be further improved by establishing optimal printing parameters (Temperature, pressure, speed).

Biological cells or biologically active molecules can be incorporated into bioinks E and F and printed in the presence of coral as the second bioink to produce the coral scaffold with some rigidity and the physical topography for biological cell attachment.

## Claims

1. A bioink comprising coral particles and biocompatible polymer, wherein the concentration of coral particles in the bioink is 25 to 85 weight % of the total weight of the bioink.

2. A bioink as claimed in claim 1, wherein the concentration of coral particles in the bioink is 30 to 80 weight % of the total weight of the bioink.

3. A bioink as claimed in claims 1 or 2, wherein the coral particles have a mean particle size of greater than 30 µm.

4. A bioink comprising coral particles, biocompatible polymer and biological cells and/or biologically active molecules, wherein the concentration of coral particles in the bioink is 1 to 25 weight % of the total weight of the bioink.

5. A bioink as claimed in claim 4, wherein the concentration of coral particles in the bioink is 1 to 20 weight % of the total weight of the bioink.

6. A bioink as claimed in claim 4 or 5, wherein the biological cells are selected from osteoblasts, stem cells, coral zygotes, coral planulae and zooxanthellae.

7. A bioink as claimed in any one of claims 4 to 6, wherein the biologically active molecules are selected from growth factors and nutrients.

8. A bioink as claimed in any one of claims 4 to 7, wherein the coral particles have a mean particle size of less than 35 µm.

9. A bioink as claimed in any one of the preceding claims, wherein the coral particles are of the genus selected from *Pocillopora, Montipora, Favites, Favia, Porites, Goniastrea, Acanthestrea, Acropora, Alvepora, Fungia, Galaxea, Hydnophora, Millepora, Seriatopora, Stylophora* and/or *Scleractinia.*

10. A bioink as claimed in any one of the preceding claims, wherein the biocompatible polymer comprises a crosslinkable polymer.

11. A bioink as claimed in claim 10, wherein the crosslinkable polymer is selected from alginate, methacrylated alginate, methacrylated collagen and methacrylated gelatin.

12. A kit comprising a bioink as claimed in claim 1, and a bioink as claimed in claim 4.

13. A method of producing a coral scaffold, said method comprising
producing a first portion of the coral scaffold by extruding a first bioink comprising coral particles and a biocompatible polymer, wherein the concentration of coral particles in the first bioink is 25 to 80 weight % of the total weight of the first bioink; and/or
producing a second portion of the coral scaffold by extruding a second bioink comprising coral particles, a biocompatible polymer and biological cells and/or biologically active molecules, wherein the concentration of coral particles in the second bioink is 1 to 25 weight % of the total weight of the second bioink;
said method further comprising crosslinking the biocompatible polymer in the first portion and/or second portion of the scaffold.

14. A method as claimed in claim 13, which comprises producing the first portion to provide a supporting structure of the scaffold, and which comprises producing the second portion to form a coating on at least part of the supporting structure.

15. A method as claimed in claims 13 or 14, wherein the coral scaffold is used as or to form a coral tissue model, or wherein the coral scaffold is used to produce a bone tissue model for testing efficacy of orthopedic treatments.

16. A coral scaffold comprising a first portion that comprises coral and a biocompatible polymer, wherein the concentration of coral in the first portion is 25 to 80 weight % of the total weight of the first portion; and/or
a second portion that comprises coral, a biocompatible polymer and biological cells and/or biologically active molecules, wherein the concentration of coral in the second portion is 1 to 25 weight % of the second portion.

17. A method of growing coral, said method comprising
extruding a bioink comprising coral particles and biocompatible polymer to form a coral scaffold precursor,
crosslinking the coral scaffold precursor to form the coral scaffold;
positioning the coral scaffold in a growth medium, and
culturing coral growth over the coral scaffold.

18. A method as claimed in claim 17, wherein the bioink contains 1 to 85 weight % coral particles .

19. A method as claimed in claim 17 or 18, wherein bioink further comprises at least one of coral zygotes, coral planulae, zooxanthellae and/or growth factors.

20. A method as claimed in any one of claims 17 to 19, wherein coral growth is cultured over the coral scaffold by introducing coral zygotes (fertilized eggs) and/or coral planulae (coral larvae) and/or zooxanthellae (coral-symbiotic single celled algae) into the growth medium.

21. A method as claimed in any one of claims 17 to 20, wherein the coral particles are of the genus selected from *Pocillopora, Montipora, Favites, Favia, Porites, Goniastrea, Acanthestrea, Acropora, Alvepora, Fungia, Galaxea, Hydnophora, Millepora, Seriatopora, Stylophora* and/or *Scleractinia.*

22. A method as claimed in any one of claims 17 to 21, wherein said method is a method of coral reef conservation.
